(19) **Europäisches Patentamt / European Patent Office / Office européen des brevets**

(11) **EP 3 543 652 B1**

(12) **FASCICULE DE BREVET EUROPEEN**

(45) Date de publication et mention de la délivrance du brevet:
**10.03.2021 Bulletin 2021/10**

(51) Int Cl.:
**G01C 21/10** (2006.01)   **H03M 1/12** (2006.01)
**A61B 5/11** (2006.01)

(21) Numéro de dépôt: **19164347.7**

(22) Date de dépôt: **21.03.2019**

(54) **PROCÉDÉ DE DÉTECTION DE PICS D'ACCÉLERATION À ECHANTILLONNAGE NON-UNIFORME**

**ERKENNUNGSVERFAHREN DER BESCHLEUNIGUNGSSPITZEN MIT NICHT-UNIFORMER PROBENAHME**

**METHOD FOR DETECTING ACCELERATION PEAKS BY NON-UNIFORM SAMPLING**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.03.2018 FR 1852448**

(43) Date de publication de la demande:
**25.09.2019 Bulletin 2019/39**

(73) Titulaire: **Commissariat à l'énergie atomique et aux énergies alternatives 75015 Paris (FR)**

(72) Inventeur: **LEIRENS, Sylvain 38054 Grenoble Cedex 09 (FR)**

(74) Mandataire: **Brevalex 95, rue d'Amsterdam 75378 Paris Cedex 8 (FR)**

(56) Documents cités:
**US-A1- 2008 238 423    US-A1- 2012 257 698
US-A1- 2015 112 218    US-A1- 2017 122 769**

- **SAMULI HEMMINKI ET AL: "Accelerometer-based transportation mode detection on smartphones", PROCEEDINGS OF THE 11TH ACM CONFERENCE ON EMBEDDED NETWORKED SENSOR SYSTEMS, SENSYS '13, 1 janvier 2013 (2013-01-01), pages 1-14, XP055535579, New York, New York, USA DOI: 10.1145/2517351.2517367 ISBN: 978-1-4503-2027-6**
- **"Nonuniform sampling", Wikipedia , 8 décembre 2016 (2016-12-08), XP002787523, Extrait de l'Internet: URL:https://en.wikipedia.org/w/index.php?title=Nonuniform_sampling&oldid=753620216 [extrait le 2019-01-02]**

**Description**

**DOMAINE TECHNIQUE**

Le domaine de l'invention est celui de la détermination d'informations relatives au déplacement d'un objet à partir de mesures délivrées par un accéléromètre. L'invention vise plus particulièrement la reconnaissance automatique d'une modalité de déplacement de l'objet au moyen d'une détection de pics d'accélération et d'une caractérisation de ces pics pour identifier un profil d'accélération typique d'un mode de transport.

**ÉTAT DE LA TECHNIQUE ANTÉRIEURE**

Afin de pouvoir identifier automatiquement le mode de transport utilisé par l'utilisateur d'un terminal mobile multifonction, il est connu de venir identifier un profil d'accélération (durée, amplitude et fréquence des pics d'accélération) caractéristique d'un mode de transport. On pourra par exemple se référer à l'article de S. Hemminki, P. Nurmi, et S. Tarkoma, "Accelerometer-based transportation mode detection on smartphones", Proceedings of the 11th ACM Conférence on Embedded Networked Sensor Systems 2013, pp. 1-14, qui propose une détection des pics d'accélération par franchissement d'un seuil sur la norme de l'accélération horizontale.

Une telle approche nécessite d'acquérir des mesures d'un accéléromètre sur des fenêtres temporelles relativement longues, pouvant s'étendre jusqu'à plusieurs minutes pour discriminer un transport en métro d'un transport en tramway. Or les systèmes embarqués ont en général une capacité de stockage limitée.

**EXPOSÉ DE L'INVENTION**

L'invention a pour objectif de proposer une technique autorisant une compression des données utiles à l'extraction des caractéristiques des pics d'accélération, sans pour autant dégrader les performances de la reconnaissance automatique de la modalité de déplacement. Elle propose pour ce faire un procédé de détermination d'informations relatives au déplacement d'un objet à partir de mesures délivrées par un accéléromètre associé à l'objet. Le procédé comprend des étapes de détection de pics d'accélération dans les mesures, de calcul d'une ou plusieurs caractéristiques des pics d'accélération détectés et de détermination d'une modalité de déplacement de l'objet à partir de la ou des caractéristiques calculées. Le procédé comprend, avant l'étape de détection des pics d'accélération, une étape d'échantillonnage non uniforme des mesures. L'étape d'échantillonnage non uniforme des mesures comprend un échantillonnage régulier des mesures, la détection d'extrema locaux dans les mesures régulièrement échantillonnées et un ré-échantillonnage non uniforme réalisé pour conserver les extrema locaux ;

Certains aspects préférés mais non limitatifs de ce procédé sont les suivants :

- le ré-échantillonnage non uniforme est en outre réalisé pour conserver un échantillon lorsque la durée séparant ledit échantillon d'un échantillon préalablement conservé est supérieure à un seuil temporel ;
- le ré-échantillonnage non uniforme est en outre réalisé pour conserver un échantillon lorsque la variation d'amplitude entre ledit échantillon et un échantillon préalablement conservé est supérieure à un seuil d'amplitude ;
- le ré-échantillonnage non uniforme est en outre réalisé pour conserver des échantillons aléatoirement sélectionnés ;
- la détection d'extrema locaux est réalisée sur une fenêtre glissante couvrant une pluralité d'échantillons successifs ;
- la détection d'extrema locaux comprend en outre la mémorisation du type d'extremum du dernier extremum détecté ;
- la détection d'extrema locaux comprend la corrélation des échantillons régulièrement échantillonnés avec une ou plusieurs formes d'onde prédéterminées, et la détection d'un extremum local en cas d'identification d'un pic de corrélation ;
- l'étape de détection des pics d'accélération comprend la détermination d'une direction du déplacement de l'objet, la détermination d'un seuil de détection de pic d'accélération à partir des mesures non uniformément échantillonnées projetées sur la direction du déplacement de l'objet et la comparaison de l'amplitude des mesures non uniformément échantillonnées au seuil de détection de pic d'accélération ;
- la détermination du seuil de détection de pic d'accélération comprend le calcul de la variance des mesures non uniformément échantillonnées ;
- il comprend en outre la comparaison de l'amplitude des mesures non uniformément échantillonnées à un seuil de bruit et un pic d'accélération est constitué de mesures non uniformément échantillonnées successives dont l'amplitude est supérieure au seuil de bruit et qui incluent au moins une mesure dont l'amplitude est supérieure au seuil de détection de pic ;
- l'étape de calcul d'une ou plusieurs caractéristiques des pics d'accélération détectés comprend la détermination d'au moins une caractéristique parmi les caractéristiques suivantes : amplitude moyenne, durée moyenne et fréquence.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- la figure 1 illustre des critères d'échantillonnage non uniforme pouvant être mis en œuvre dans l'invention ;
- les figures 2a et 2b représentent le résultat d'une analyse en composantes principales réalisée pour rechercher la direction du mouvement, respectivement à partir de l'accélération propre et de l'accélération horizontale ;
- la figure 3 illustre la détection et délimitation d'un pic d'accélération ;
- les figures 4a et 4b représentent respectivement l'échantillonnage non uniforme et la détection de pics d'accélération selon l'invention dans un exemple de déplacement en métro ;
- les figures 5a et 5b représentent respectivement l'échantillonnage non uniforme et la détection de pics d'accélération selon l'invention dans un exemple de déplacement en voiture.

## EXPOSÉ DÉTAILLÉ DE MODES DE RÉALISATION PARTICULIERS

L'invention porte sur un procédé de détermination d'informations relatives au déplacement d'un objet à partir de mesures délivrées par un accéléromètre associé à l'objet. L'objet peut être un terminal mobile d'utilisateur, par exemple un téléphone multifonction.

Le procédé selon l'invention comprend les étapes de détection de pics d'accélération dans les mesures, de calcul d'une ou plusieurs caractéristiques des pics d'accélération détectés et de détermination d'une modalité de déplacement de l'objet à partir de la ou des caractéristiques calculées. L'invention propose, avant l'étape de détection des pics d'accélération, de mettre en œuvre une étape d'échantillonnage non uniforme des mesures.

L'étape d'échantillonnage non uniforme des mesures comprend un échantillonnage régulier des mesures, la détection d'extrema locaux dans les mesures régulièrement échantillonnées et un ré-échantillonnage non uniforme réalisé pour conserver les extrema locaux.

Selon l'exemple de réalisation illustrant le procédé selon l'invention, un accéléromètre de type capteur tri-axe est utilisé et les mesures issues de cet accéléromètre sont alors de dimensions 3 (3 composantes d'accélérations). Dans un premier mode de réalisation, on peut déterminer quel est l'axe de variance maximale et réaliser la détection d'extrema locaux dans la composante d'accélération correspondant à cet axe de variance maximale. Dans un second mode de réalisation, la détection d'extrema locaux peut être réalisée dans chacune des 3 composantes d'accélération, la détection d'un extremum local dans l'une des composantes entrainant le ré-échantillonnage sur les deux autres composantes. Ce second mode de réalisation résulte en un nombre d'échantillons plus important.

D'autres conditions peuvent être ajoutées pour ré-échantillonner davantage de points, notamment un échantillonnage aléatoire ou des conditions de variation suffisante du signal ou de temps écoulé. Ainsi le ré-échantillonnage non uniforme peut en outre être réalisé pour conserver un échantillon lorsque la durée séparant ledit échantillon d'un échantillon préalablement conservé est supérieure à un seuil temporel $\Delta t^{max}$. Il peut également être réalisé pour conserver un échantillon lorsque la variation d'amplitude entre ledit échantillon et un échantillon préalablement conservé est supérieure à un seuil d'amplitude $\Delta x^{max}$. Et il peut aussi être réalisé pour conserver des échantillons aléatoirement sélectionnés.

La détection des extrema locaux peut être réalisée sur une fenêtre glissante couvrant une pluralité d'échantillons successifs des mesures d'accélération et comprendre le calcul d'un ou plusieurs taux d'accroissement entre échantillons de la fenêtre.

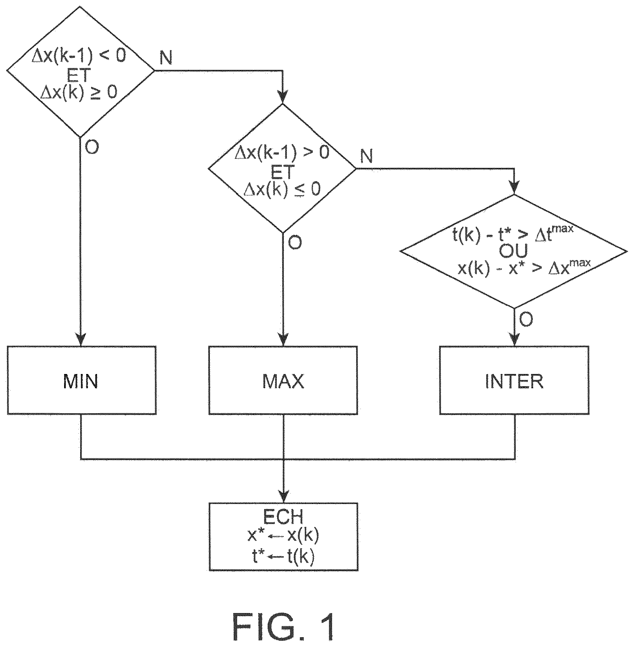

Supposons une composante d'accélération x échantillonnée aux instants $t(k)$ repérés par l'index $k$. La détection des extrema locaux est par exemple réalisée sur une fenêtre glissante couvrant trois échantillons successifs $k - 1$, $k$ et $k + 1$. On calcule des taux d'accroissement successifs $\Delta x(k - 1) = x(k) - x(k - 1)$ et $\Delta x(k) = x(k + 1) - x(k)$ pour déterminer si l'échantillon central $k$ est au sommet d'un triangle formé par les trois échantillons et constitue ainsi un extremum local. Plus particulièrement, comme représenté en figure 1, si $\Delta x(k - 1) < 0$ et $\Delta x(k) \geq 0$, alors l'échantillon $k$ est un minimum local MIN et si $\Delta x(k - 1) > 0$ et $\Delta x(k) \leq 0$, alors l'échantillon $k$ est un maximum local MAX. Lorsqu'un extremum local est ainsi détecté, son amplitude et son instant peuvent être mémorisés respectivement en tant que $x^*$ et $t^*$ pour permettre la mise en œuvre du ré-échantillonnage sous conditions de variation suffisante du signal et de temps écoulé. De plus, comme représenté sur la figure 1, si $t(k) - t^* > \Delta t^{max}$ ou si $(k) - x^* > \Delta x^{max}$, alors l'échantillon $k$ est repéré comme échantillon intermédiaire INTER et est inclus dans le ré-échantillonnage, son amplitude et son instant sont mémorisées.

On peut par ailleurs adopter un seuil portant sur une durée minimale entre deux échantillons $\Delta t^{min}$ et/ou un seuil portant sur une amplitude minimale entre deux échantillons $\Delta x^{min}$ pour ne pas déclencher le ré-échantillonnage tant que la durée écoulée depuis le dernier échantillon mémorisé est inférieure à $\Delta t^{min}$ et/ou tant que la variation d'amplitude du signal n'a pas atteint $\mathrm{A}x^{min}$.

Dans un mode de réalisation possible, la détection d'extrema locaux comprend en outre la mémorisation du type d'extremum du dernier extremum détecté, à savoir s'il s'agit d'un maximum ou un minimum local. De telle manière, il n'est nécessaire de ne calculer qu'un seul taux d'accroissement $\Delta x(k) = x(k + 1) - x(k)$. En effet, un maximum (respectivement un minimum) est identifié à l'échantillon $k$ si le dernier extremum identifié est un minimum (respectivement un maximum) et que le taux d'accroissement est négatif (respectivement positif).

Dans une variante de réalisation, la détection d'extrema locaux comprend la corrélation des échantillons régulièrement échantillonnés avec une ou plusieurs formes d'onde prédéterminées, et la détection d'un extremum local en cas d'identification d'un pic de corrélation. La ou les formes d'ondes sont typiquement représentatives des formes des pics d'accélération attendues pour les différentes modalités de transport.

Une fois le ré-échantillonnage réalisé, le procédé selon l'invention met en œuvre une étape de détection des pics d'accélération à partir des mesures non uniformément échantillonnées.

Cette étape de détection des pics d'accélération comprend la détermination d'une direction du déplacement de l'objet. Supposons que M échantillons résultent de l'échantillonnage non uniforme. On estime la gravité en calculant la moyenne du signal sur la fenêtre temporelle : $\hat{g} = \frac{1}{M} \sum_{i=1}^{M} a_{\mathrm{m}}(i),$ où $a_{\mathrm{m}}(i)$ est le i-ème échantillon ré-échantillonné de la mesure d'accélération (de dimension 3). En ôtant la gravité estimée de l'accélération mesurée, on obtient une estimation de l'accélération propre de l'objet : $\hat{a}(i) = a_{\mathrm{m}}(i) - \hat{g}$.

La direction du mouvement peut alors être déterminée en effectuant une analyse en composantes principales de l'accélération propre $\hat{a}$ et en faisant l'hypothèse que la direction du mouvement est celle de variance maximale.

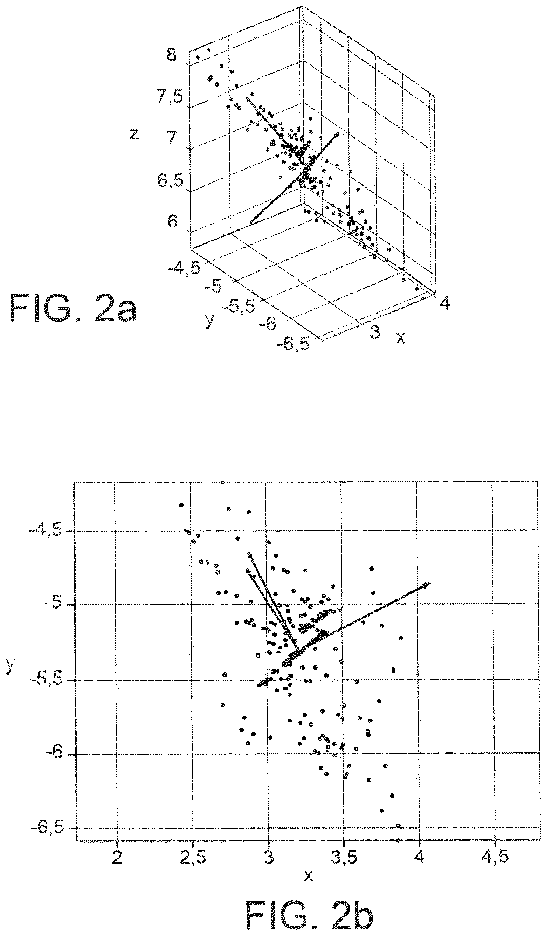

Pour réaliser cette analyse en composantes principes, on commence par projeter l'accélération propre $\hat{a}$ dans le plan horizontal, en d'autres termes, on vient ôter la composante verticale de l'accélération propre: $a_{\mathrm{h}}(i) = \hat{a}(i) - \hat{a}(i)^T v$, où $v$ est un vecteur unitaire opposé à $\hat{g}$ (i.e. $v = -\hat{g}/||\hat{g}||$). Si l'accélération horizontale $a_{\mathrm{h}}$ est un vecteur de dimension 3, ses échantillons sont contenus dans un sous-espace de dimension 2 (i.e. contenus dans le plan horizontal). On réalise une analyse en composantes principales de l'accélération horizontale. La direction de variance maximale $h_1$ est donnée par le vecteur propre correspondant à la valeur propre maximale de la matrice de corrélation de l'accélération horizontale (matrice 3x3). On a représenté sur les figures 2a et 2b le résultat d'une analyse en composantes principales réalisée pour rechercher la direction du mouvement, respectivement à partir de l'accélération propre et de l'accélération horizontale.

Cette analyse repose sur l'estimation de la gravité qui peut être problématique. L'hypothèse que l'accélération propre est nulle en moyenne sur la fenêtre temporelle n'est pas toujours facile à satisfaire en pratique. Une approche alternative est d'effectuer l'analyse en composantes principales sur l'accélération mesurée $a_{\mathrm{m}}$. Le problème de l'estimation de la gravité ne se pose plus, mais l'hypothèse que la direction du mouvement est celle de variance maximale est plus difficile à vérifier, surtout en présence de bruits ou de vibrations (notamment selon l'axe vertical).

Une fois la direction du mouvement identifiée comme étant selon le premier axe principal $h_1$, on projette l'accélération horizontale (ou l'accélération propre suivant la méthode choisie) selon $h_1$ afin d'obtenir une accélération longitudinale $a_{\mathrm{h1}}$ correspondant aux mesures non uniformément échantillonnées projetées sur la direction du déplacement de l'objet. L'étape de détection des pics d'accélération se poursuit avec la détermination, à partir de l'accélération longitudinale $a_{\mathrm{h1}}$, d'un seuil adaptatif de détection de pic d'accélération. La détermination de ce seuil peut notamment comprendre le calcul de la variance des mesures non uniformément échantillonnées.

Pour simplifier les notations, on renomme x l'accélération longitudinale $a_{\mathrm{h1}}$. La variance empirique $\sigma^2$ sur une fenêtre de M échantillons d'un signal x échantillonné irrégulièrement est calculée comme suit :

$$\sigma^2 = \frac{1}{t(M) - t(1)} \sum_{i=2}^{M} (x(i) - \mu)^2 (t(i) - t(i-1))$$

où la moyenne empirique $\mu$ est donnée par :

$$\mu = \frac{1}{t(M) - t(1)} \sum_{i=2}^{M} x(i) (t(i) - t(i-1))$$

Le seuil de détection de pic d'accélération peut être calculé sur la base de la largeur à mi-hauteur H de la loi normale qui est proportionnelle à $\sigma$ : $H = 2\sqrt{2\ln 2}\,\sigma,$ et le seuil s est pris égal à la moitié de $H$ : $s = H/2$.

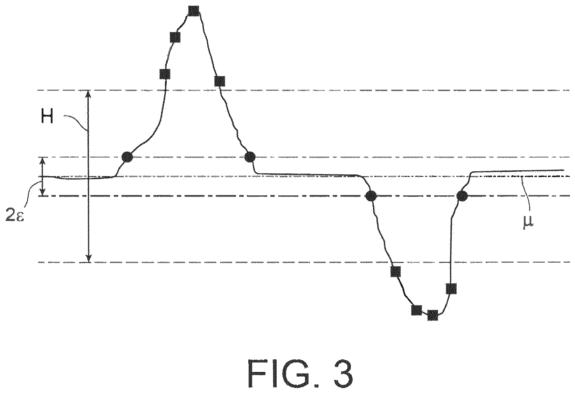

On a représenté sur la figure 3 la détection et la délimitation d'un pic d'accélération. La détection d'un pic d'accélération comprend la comparaison de l'amplitude des mesures non uniformément échantillonnées au seuil de détection de pic d'accélération s et procède par l'identification des échantillons se trouvant en dehors de la zone de la largeur H (échantillons représentés par des carrés sur la figure 3).

La délimitation d'un pic d'accélération comprend la comparaison de l'amplitude des mesures non uniformément échantillonnées à un seuil de bruit $\varepsilon$. Un pic d'accélération est alors constitué de mesures non uniformément échantillonnées successives dont l'amplitude est supérieure au seuil de bruit $\varepsilon$ et qui incluent au moins une mesure dont l'amplitude est supérieure au seuil de détection de pic s. Le recours à un seuil de bruit rend la délimitation des pics d'accélération robuste au bruit.

Comme représenté sur la figure 3, le seuil de bruit $\varepsilon$ peut être une tolérance autour de la moyenne empirique $\mu$, par exemple 0,1 m/s$^2$. Le début et la fin d'un pic sont alors déterminés en recherchant les échantillons au plus proche de la zone de largeur $2\varepsilon$ autour de la moyenne empirique $\mu$ (échantillons représentés par des cercles sur la figure 3).

Une fois les pics d'accélération détectés et délimités, le procédé comprend le calcul d'une ou plusieurs caractéristiques de chaque pic d'accélération en vue, par exemple, d'une classification automatique des modes de transports. On vient par exemple déterminer au moins une caractéristique parmi les caractéristiques suivantes : amplitude moyenne, durée moyenne et fréquence des pics d'accélération. La classification automatique peut être réalisée au moyen d'algorithmes tels que les arbres de décision ou les forêts d'arbres aléatoires.

L'invention n'est pas limitée au procédé tel que précédemment décrit, mais s'étend également à un programme d'ordinateur comprenant des instructions pour l'exécution de ce procédé lorsque ledit programme est exécuté sur un ordinateur. Et l'invention s'étend également à un dispositif, par exemple un dispositif embarqué au sein d'un terminal mobile d'utilisateur, comprenant une unité de traitement de données configurée pour mettre en œuvre ce procédé.

Il découle de ce qui précède que l'invention propose notamment un ré-échantillonnage sur détection d'extrema de l'axe de variance maximale ou couplé sur les 3 axes. Pour plus de robustesse, ce ré-échantillonnage peur être enrichi par détection de seuils et/ou par un échantillonnage aléatoire. L'invention propose également de venir estimer l'accélération longitudinale et de calculer des caractéristiques du profil d'accélération longitudinale au moyen d'une détection de pics d'accélération par franchissement d'un seuil adaptatif. L'invention présente ainsi l'avantage d'autoriser une compression des données adaptée au contenu. Elle ne nécessite pas de reconstruction du signal pour l'extraction des caractéristiques, est simple d'implémentation et peut faire l'objet de réglages simples.

On présente dans ce qui suit deux exemples de mise en œuvre de l'invention. Dans chacun de ces exemples, on adopte les réglages suivants : $\Delta t^{max}$ = 5 s, $\Delta x^{max}$ = 0,2 m/s$^2$, $\Delta t^{mm}$ = 0 s et $\Delta x^{min}$ = 0,02 m/s$^2$.

Le premier exemple correspond à un déplacement en métro. La fréquence d'échantillonnage de l'accéléromètre tri-axes est de 100 Hz, la fenêtre temporelle considérée est de 60 secondes, le nombre d'échantillons est de 6000 et un filtrage passe-bas de fréquence de coupure 2 Hz à -3 dB est réalisé sur les mesures brutes délivrées par l'accéléromètre.

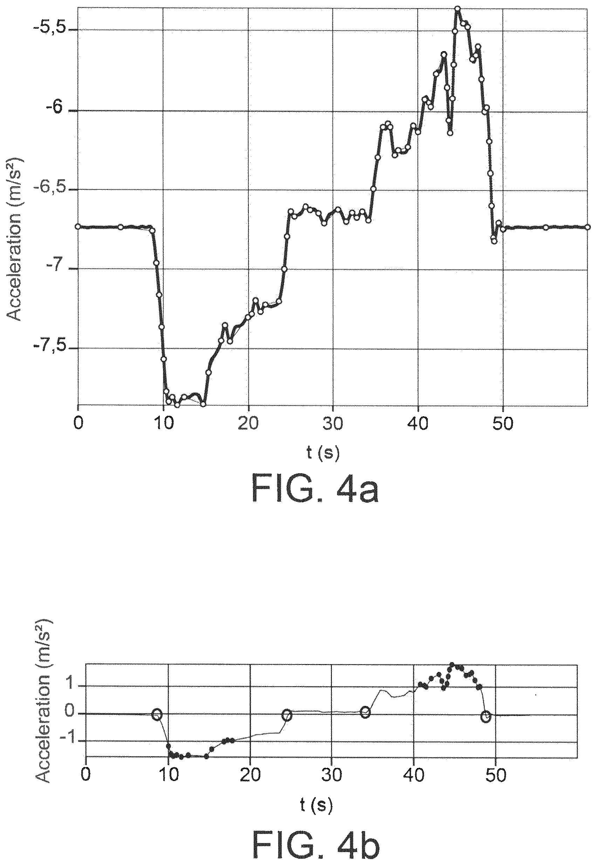

La figure 4a illustre le ré-échantillonnage non uniforme de la composante du signal d'accéléromètre correspondant à l'axe de variance maximale. 76 échantillons sont conservés, la fréquence de ré-échantillonnage moyenne étant de 1,25Hz. La figure 4b illustre la détection des pics d'accélération. Le seuil de détection calculé selon l'exemple de réalisation présenté ci-dessus est égal à 0,924m/s$^2$. L'amplitude moyenne des deux pics détectés (amplitude moyenne des échantillons au-dessus du seuil de détection pour les amplitudes positives et amplitude moyenne des échantillons en dessous de l'opposé du seuil de détection pour les amplitudes négatives) est de 1,01m/s$^2$ et de -1,04m/s$^2$, à comparer à des amplitudes de référence de 1,02m/s$^2$ et de -1,05m/s$^2$ telles que calculées avec un échantillonnage régulier. La durées des pics détectés est de 15,7s et 14,4s, à comparer à des durées de référence de 15,6s et de 14,3s telles que calculées avec un échantillonnage régulier. La fréquence des pics détectés est de 0,0333Hz soit 2,0 min$^{-1}$, à comparer à une fréquence de référence de 2,0min$^{-1}$ telle que calculée avec un échantillonnage régulier.

Le deuxième exemple correspond à un déplacement en voiture. La fréquence d'échantillonnage de l'accéléromètre tri-axes est de 86,5 Hz, la fenêtre temporelle considérée est de 30s, le nombre d'échantillons est de 2566 et un filtrage passe-bas de fréquence de coupure 2 Hz à -3 dB est réalisé sur les mesures brutes délivrées par l'accéléromètre.

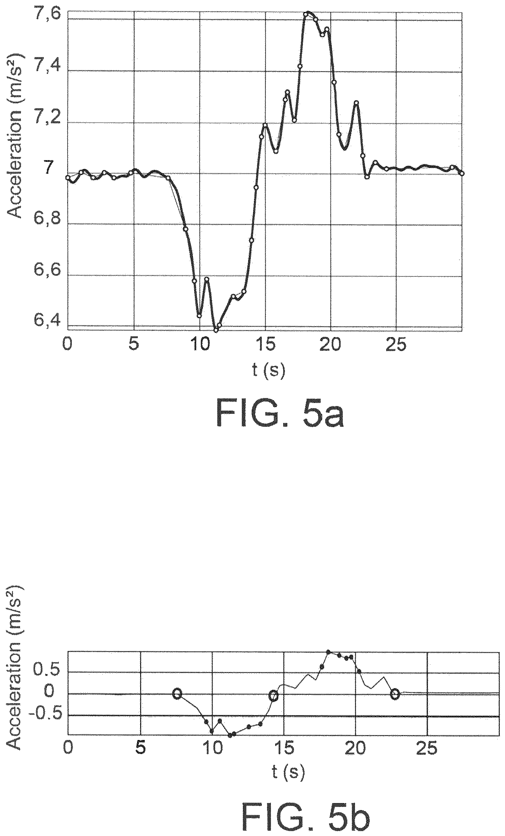

La figure 5a illustre le ré-échantillonnage non uniforme de la composante du signal d'accéléromètre correspondant à l'axe de variance maximale. 39 échantillons sont conservés, la fréquence de ré-échantillonnage moyenne étant de 1,26Hz. La figure 5b illustre la détection des pics d'accélération. Le seuil de détection calculé selon l'exemple de réalisation présenté ci-dessus est égal à 0,490m/s$^2$. L'amplitude moyenne des deux pics détectés est de -0,60m/s$^2$ et de 0,44m/s$^2$, à comparer à des amplitudes de référence de -0,63m/s$^2$ et de 0,46m/s$^2$ telles que calculées avec un échantillonnage régulier. La durées des pics détectés est de 6,24s et 7,95s, à comparer à des durées de référence de 6,04s et de 7,97s telles que calculées avec un échantillonnage régulier. La fréquence des pics détectés est de 0,0667Hz soit 4,0 min$^{-1}$, à comparer à une fréquence de référence de 4,0min$^{-1}$ telle que calculée avec un échantillonnage régulier.

**Revendications**

1. Procédé de détermination d'informations relatives au déplacement d'un objet à partir de mesures délivrées par un accéléromètre associé à l'objet, comprenant des étapes de détection de pics d'accélération dans les mesures, de calcul d'une ou plusieurs caractéristiques des pics d'accélération détectés et de détermination d'une modalité de déplacement de l'objet à partir de la ou des caractéristiques calculées, **caractérisé en ce qu'**il comprend, avant l'étape de détection des pics d'accélération, une étape d'échantillonnage non uniforme des mesures (ECH) qui comprend un échantillonnage régulier des mesures, la détection d'extrema locaux (MIN, MAX) dans les mesures régulièrement échantillonnées et un ré-échantillonnage non uniforme des mesures réalisé pour conserver les extrema locaux détectés.

2. Procédé selon la revendication 1, dans lequel le ré-échantillonnage non uniforme est en outre réalisé pour conserver un échantillon (INTER) lorsque la durée séparant ledit échantillon d'un échantillon préalablement conservé est supérieure à un seuil temporel.

3. Procédé selon l'une des revendications 1 et 2, dans lequel le ré-échantillonnage non uniforme est en outre réalisé pour conserver un échantillon (INTER) lorsque la variation d'amplitude entre ledit échantillon et un échantillon préalablement conservé est supérieure à un seuil d'amplitude.

4. Procédé selon l'une des revendications 1 à 3, dans lequel le ré-échantillonnage non uniforme est en outre réalisé pour conserver des échantillons aléatoirement sélectionnés.

5. Procédé selon l'une des revendications 1 à 4, dans lequel la détection d'extrema locaux est réalisée sur une fenêtre glissante couvrant une pluralité d'échantillons successifs.

6. Procédé selon la revendication 5, dans lequel la détection d'extrema locaux comprend en outre la mémorisation du type d'extremum du dernier extremum détecté.

7. Procédé selon l'une des revendications 1 à 4, dans lequel la détection d'extrema locaux comprend la corrélation des échantillons régulièrement échantillonnés avec une ou plusieurs formes d'onde prédéterminées, et la détection d'un extremum local en cas d'identification d'un pic de corrélation.

8. Procédé selon l'une des revendications 1 à 7, dans lequel l'étape de détection des pics d'accélération comprend la détermination d'une direction du déplacement de l'objet, la détermination d'un seuil de détection (H/2) de pic d'accélération à partir des mesures non uniformément échantillonnées projetées sur la direction du déplacement de l'objet et la comparaison de l'amplitude des mesures non uniformément échantillonnées au seuil de détection de pic d'accélération.

9. Procédé selon la revendication 8, dans lequel la détermination du seuil de détection de pic d'accélération comprend le calcul de la variance des mesures non uniformément échantillonnées.

10. Procédé selon l'une des revendications 8 et 9, comprenant en outre la comparaison de l'amplitude des mesures non uniformément échantillonnées à un seuil de bruit ($\varepsilon$) et dans lequel un pic d'accélération est constitué de mesures non uniformément échantillonnées successives dont l'amplitude est supérieure au seuil de bruit et qui incluent au moins une mesure dont l'amplitude est supérieure au seuil de détection de pic.

11. Procédé selon l'une des revendications 1 à 10, dans lequel l'étape de calcul d'une ou plusieurs caractéristiques des pics d'accélération détectés comprend la détermination d'au moins une caractéristique parmi les caractéristiques suivantes : amplitude moyenne, durée moyenne et fréquence.

12. Programme d'ordinateur comprenant des instructions pour l'exécution du procédé selon l'une des revendications 1 à 11 lorsque ledit programme est exécuté sur un ordinateur.

13. Dispositif comprenant une unité de traitement de données configurée pour mettre en œuvre le procédé selon l'une des revendications 1 à 11.

**Patentansprüche**

1. Verfahren zum Bestimmen von Informationen bezüglich der Bewegung eines Objekts aus Messungen, die von einem dem Objekt zugeordneten Beschleunigungsmesser bereitgestellt werden, umfassend die Schritte des Erfassens von Beschleunigungsspitzen bei den Messungen, des Berechnens eines oder mehrerer Merkmale der erfassten Beschleunigungsspitzen und des Bestimmens einer Bewegungsmodalität des Objekts aus dem berechneten Merkmal oder den berechneten Merkmalen,
dadurch gekennzeichnet, dass es vor dem Schritt des Erfassens der Beschleunigungsspitzen einen Schritt des ungleichförmigen Abtastens der Messungen (ECH) umfasst, der das regelmäßige Abtasten der Messungen, das Erfassen lokaler Extremwerte (MIN, MAX) bei den regelmäßig abgetasteten Messungen und ein ungleichförmiges Neuabtasten der Messungen umfasst, das durchgeführt wird, um die erfassten lokalen Extremwerte zu hinterlegen.

2. Verfahren nach Anspruch 1, wobei das ungleichförmige Neuabtasten ferner durchgeführt wird, um eine Abtastung (INTER) zu hinterlegen, wenn die Zeitdauer, die die Abtastung von einer zuvor hinterlegten Abtastung trennt, größer als ein Zeitschwellenwert ist.

3. Verfahren nach einem der Ansprüche 1 und 2, wobei das ungleichförmige Neuabtasten ferner durchgeführt wird, um eine Abtastung (INTER) zu hinterlegen, wenn die Amplitudenschwankung zwischen der Abtastung und einer zuvor hinterlegten Abtastung größer als ein Amplitudenschwellenwert ist.

4. Verfahren nach einem der Ansprüche 1 bis 3, wobei das ungleichförmige Neuabtasten ferner durchgeführt wird, um zufällig ausgewählte Abtastungen zu vieren.

5. Verfahren nach einem der Ansprüche 1 bis 4, wobei das Erfassen lokaler Extremwerte über ein Gleitfenster durchgeführt wird, das eine Vielzahl von aufeinanderfolgenden Abtastungen abdeckt.

6. Verfahren nach Anspruch 5, wobei das Erfassen lokaler Extremwerte ferner das Speichern des Extremwerttyps des zuletzt erfassen Extremwerts umfasst.

7. Verfahren nach einem der Ansprüche 1 bis 4, wobei das Erfassen lokaler Extremwerte die Korrelation von regelmäßig abgetasteten Abtastungen mit einer oder mehreren vorbestimmten Wellenformen und das Erfassen eines lokalen Extremwerts bei Identifizierung einer Korrelationsspitze umfasst.

8. Verfahren nach einem der Ansprüche 1 bis 7, wobei der Schritt des Erfassens von Beschleunigungsspitzen das Bestimmen einer Bewegungsrichtung des Objekts, das Bestimmen einer Beschleunigungsspitzen-Erfassungsschwelle (H/2) aus den auf die Bewegungsrichtung des Objekts projizierten ungleichförmig abgetasteten Messungen und das Vergleichen der Amplitude der ungleichförmig abgetasteten Messungen mit der Beschleunigungsspitzen-Erfassungsschwelle umfasst.

9. Verfahren nach Anspruch 8, wobei das Bestimmen der Beschleunigungsspitzen-Erfassungsschwelle das Berechnen der Varianz der ungleichförmig abgetasteten Messungen umfasst.

10. Verfahren nach einem der Ansprüche 8 und 9, ferner umfassend das Vergleichen der Amplitude der ungleichförmig abgetasteten Messungen mit einer Rauschschwelle ($\varepsilon$), wobei eine Beschleunigungsspitze aus aufeinanderfolgenden ungleichförmig abgetasteten Messungen besteht, deren Amplitude größer als die Rauschschwelle ist und die zumindest eine Messung umfassen, deren Amplitude größer als die Spitzenerfassungsschwelle ist.

11. Verfahren nach einem der Ansprüche 1 bis 10, wobei der Schritt des Berechnens eines oder mehrerer Merkmale von erfassten Beschleunigungsspitzen das Bestimmen von zumindest einem der folgenden Merkmale umfasst: mittlere Amplitude, mittlere Dauer und Frequenz.

12. Computerprogramm mit Anweisungen zum Durchführen der Verfahrens nach einem der Ansprüche 1 bis 11, wenn das Programm auf einem Computer ausgeführt wird.

13. Vorrichtung mit einer Datenverarbeitungseinheit, die dazu ausgelegt ist, das Verfahren nach einem der Ansprüche 1 bis 11 durchzuführen.

**Claims**

1. Method for determining information regarding the displacement of an object from measurements provided by an accelerometer associated with the object, comprising steps of detecting acceleration peaks in the measurements, of calculating one or more characteristics of the detected acceleration peaks, and of determining a mode of travel of the object from the one or more calculated characteristics, **characterised in that** it comprises, before the step of detecting acceleration peaks, a step of non-uniformly sampling the measurements (ECH), which comprises a regular sampling of the measurements, the detection of local extrema (MIN, MAX) in the measurements regularly sampled and a non-uniform resampling of the measurements carried out to keep the detected local extrema.

2. Method according to claim 1, wherein the non-uniform resampling is furthermore carried out in order to keep a sample (INTER) when the duration separating said sample from a previously kept sample is greater than a time threshold.

3. Method according to one of claims 1 and 2, wherein the non-uniform resampling is furthermore carried out in order to keep a sample (INTER) when the amplitude variation between said sample and a previously kept sample is greater than an amplitude threshold.

4. Method according to one of claims 1 to 3, wherein the non-uniform resampling is furthermore carried out in order to keep randomly selected samples.

5. Method according to one of claims 1 to 4, wherein the detection of local extrema is carried out from a sliding-window covering a plurality of successive samples.

6. Method according to claim 5, wherein the detection of local extrema further comprises storing the extremum type of the last extremum detected in memory.

7. Method according to one of claims 1 to 4, wherein the detection of local extrema comprises correlating the regularly sampled samples with one or more predetermined waveforms, and detecting a local extremum in the case a correlation peak is identified.

8. Method according to one of claims 1 to 7, wherein the step of detecting the acceleration peaks comprises determining a direction of travel of the object, determining an acceleration peak detection threshold ($H/2$) from the non-uniformly sampled measurements projected in the direction of travel of the object and comparing the amplitude of the non-uniformly sampled measurements with the acceleration peak detection threshold.

9. Method according to claim 8, wherein the determination of the acceleration peak detection threshold comprises calculating the variance of the non-uniformly sampled measurements.

10. Method according to one of claims 8 and 9, further comprising comparing the amplitude of the non-uniformly sampled measurements with a noise threshold ($\varepsilon$) and wherein an acceleration peak is formed of successive non-uniformly sampled measurements the amplitude whereof is greater than the noise threshold and which include at least one measurement the amplitude whereof is greater than the peak detection threshold.

11. Method according to one of claims 1 to 10, wherein the step of calculating one or more characteristics of the detected acceleration peaks comprises determining at least one characteristic from the following characteristics: mean amplitude, mean duration and frequency.

12. Computer program comprising instructions for executing the method according to one of claims 1 to 11, when said program is executed on a computer.

13. Device comprising a data processing unit configured to implement the method according to one of claims 1 to 11.

FIG. 1

FIG. 2a

FIG. 2b

FIG. 3

FIG. 4a

FIG. 4b

FIG. 5a

FIG. 5b

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **S. HEMMINKI ; P. NURMI ; S. TARKOMA.** Accelerometer-based transportation mode detection on smartphones. *Proceedings of the 11th ACM Conférence on Embedded Networked Sensor Systems,* 2013, 1-14